# FASCICULE DE BREVET EUROPEEN

(11) **EP 0 888 136 B1**
(45) Date de publication et mention de la délivrance du brevet: **04.06.2003**
(21) Numéro de dépôt: 97906216.3
(22) Date de dépôt: 18.02.1997
(51) Int. Cl.: A61L 9/14

(54) **BOMBE AEROSOL A PROPULSEUR LIQUEFIE POUR LE PARFUMAGE D'AMBIANCE**
AEROSOLBEHÄLTER MIT FLÜSSIGEM TREIBMITTEL ZUM PARFÜMIEREN VON UMGEBUNGSLUFT
AIR FRESHENER AEROSOL CAN CONTAINING A LIQUEFIED PROPELLANT

(30) Priorité: 19.02.1996 FR 9602215
(43) Date de publication de la demande: 07.01.1999
(73) Titulaire: Jean, Marcel, 06250 Mougins (FR)
(72) Inventeur: Jean, Marcel, 06250 Mougins (FR)
(74) Mandataire: Roman, Michel
(86) Numéro de dépôt international: FR9700299
(87) Numéro de publication internationale: WO97029790

(56) Documents cités:
- EP-A- 0 343 843
- WO-A-96/00564
- FR-A- 1 341 709
- FR-A- 1 354 159
- FR-A- 2 663 642
- GB-A- 967 335
- GB-A- 969 347
- GB-A- 2 286 405
- US-A- 3 282 776
- US-A- 3 287 214

## Description

La présente invention a pour objet une bombe aérosol à propulseur liquéfié pour le parfumage d'ambiance.

Elle est destinée à être utilisée dans tous les endroits clos, qu'il s'agisse de lieux d'habitation, de lieux de travail, de véhicules ou de tous autre lieux.

Il existe de nombreux systèmes de diffusion de parfum d'ambiance, évaporateurs utilisant des mèches ou des corps spongieux imprégnés d'un liquide, gels, parfums solidifiés, bougies parfumantes, mélanges de fleurs séchées, etc. Cependant le système le plus répandu actuellement est l'aérosol sous pression qui permet de vaporiser à l'instant désiré et à volonté un produit liquide dans l'air ambiant.

Le parfumage de l'ambiance remonte à la plus haute antiquité, probablement plusieurs millénaires, puisqu'on le retrouve aussi bien en Chine, qu'en Egypte, comme plus tard à Rome, au moyen âge en France, etc.

Le parfumage corporel, directement sur la peau est réellement apparu avec l'eau de Cologne, que l'on a attribuée à Jean Marie Farina, et qui a été le premier produit vulgarisant l'utilisation des bases parfumantes en solution alcoolique.

La grande démocratisation du marché du parfum corporel, est intervenue à une époque récente au cours de la décennie 1970 avec les systèmes de pulvérisation d'aérosols.

En effet les aérosols ont apporté un progrès décisif dans la fonction de parfumage corporel en améliorant la dispersion du principe actif constitué de bases parfumantes nobles, en concentration élevée, dans de l'alcool de parfumerie, par l'utilisation de gaz propulseur de la catégorie des Chloro Fluoro Carbures (CFC 11 - 12 - 113 - 114), considérés à juste titre, comme idéal pour cette utilisation. Le mélange le plus utilisé, le CFC 12 - 114 dans la proportion 10/90, était parfaitement respectueux des qualités des bases parfumantes les plus fragiles, non inflammable et reconnu sans danger pour l'utilisation cosmétique ; cet équilibre n'a jamais été égalé depuis, quelque que soit le système qui lui a été substitué, pour répondre à la disparition des chloro fluoro carbures du marché des aérosols.

Pour la maison comme pour la voiture, la recherche de toujours plus de confort et d'agrément va instituer une utilisation régulière de systèmes déosodorisants-parfumants, ou déoparfumants, capables d'apporter à l'instant désiré et à volonté, l'atmosphère souhaitée par l'utilisateur, qu'il s'agisse de parfumage ou de déoparfumage, éventuellement de purification, d'aseptisation de l'air, ou encore d'apporter d'autres bénéfices aux consommateurs grâce à des fonctions tonifiantes, euphorisantes, relaxantes, etc..

Il est remarquable de noter que le parfumage de l'ambiance dont on trouve la trace constante depuis plusieurs millénaires a été occultée depuis le début du siècle par la recherche de désinfection, de purification absolument prioritaires pour des raisons sanitaires évidentes.

Plus récemment le mieux-être corporel sublimé par le parfumage corporel, objet de tous les soins des grands parfumeurs dans leur stratégie de développement a bien évidemment été satisfait en priorité.

Le désir de confort dans l'ambiance, de confort olfactif autour de soi, ne peut intervenir, c'est bien évident, que lorsque tous les besoins d'équipement sont pleinement satisfaits dans la voiture, comme dans la maison. Dans les pays développés, ces conditions sont aujourd'hui remplies et ceci dans la voiture comme dans la maison, souvent d'ailleurs en premier lieu dans la voiture.

Pour parfumer l'ambiance on a recours à la dispersion de produits volatils dans l'air ambiant. Dans l'état actuel de la technique, la bombe aérosol est le système le plus performant pour répondre aux attentes des consommateurs. Le parfum corporel a en effet montré que la vaporisation était de très loin la meilleure façon de ressentir un parfum. L'utilisateur peut décider du moment de l'utilisation à volonté pour son bien-être, son confort, celui de ses hôtes à la maison, de ses passagers dans la voiture.

La bombe aérosol permet de disposer d'un produit de qualité égale du début jusqu'à la fin et c'est le système le plus économique à la fabrication. Enfin, il s'agit d'un procédé pouvant s'avérer écologique tout en offrant un maximum de sécurité pour la santé humaine si l'on a pris soin d'associer :
- un récipient en aluminium 100 % recyclable,
- des bases parfumantes aux normes de la parfumerie en solution dans de l'alcool également de qualité parfumerie,
- un principe désodorisant 100 % végétal,
- et un propulseur tout à la fois non inflammable, non toxique et sans effets sur la couche d'ozone.

Pour toutes ces raisons la meilleure technique de parfumage de l'ambiance de qualité est un pulvérisateur d'aérosol avec propulseur liquéfié.

Certes la vaporisation par un aérosol nécessite une intervention pour l'activer, mais présente sur les systèmes de diffusion permanents : mèche, papier ou autres matières tampons imprégnées, gels, etc, l'avantage de ne pas disperser dans l'air quasi inutilement une forte part des principes actifs, compte tenu du renouvellement constant de l'air ambiant.

Enfin, la vaporisation sous forme de micro-gouttelettes qui peuvent se fixer et diffuser comme le font tous les vrais parfums corporels, présente un avantage déterminant sur les émanations de vapeurs qui se développent en suspension dans l'air et s'évacuent avec le renouvellement de l'air.

Les gouttelettes à condition d'être très finement atomisées vont se disperser de façon très large dans le volume (d'une pièce, d'une voiture) à traiter, et se fixer sur toutes les surfaces, en particulier les tissus, plastiques, moquettes, sièges, là où s'imprègnent généralement le mieux les odeurs. Les vaporisation successives installent progressivement et durablement les senteurs dans l'ambiance, exactement au goût et à la volonté de l'utilisateur, enfin si l'on a pris soin de n'utiliser que des bases parfums de grande qualité, il n'y aura jamais, dans le temps, de senteurs résiduelles parasites indésirables.

La demande de brevet N° FR 92 00 227 du même inventeur décrit un système aérosol élaboré partiellement selon ces principes. Il est constitué d'un récipient vaporisateur contenant un gaz propulseur liquéfié de type chlorodifluorométhane (type HFA 22 de marque ATOCHEM), ainsi qu'une ou plusieurs bases parfumantes en solution dans l'alcool, éventuellement associées à un ou plusieurs principes actifs tels que désodorisant, désinfectant, relaxant ou autre, ces bases parfumantes et principes actifs étant totalement ou partiellement emprisonnés dans des microcapsules. Ce dispositif est surtout destiné à obtenir un effet de longue durée, et son prix de revient est bien sûr grevé par la micro-encapsulation des produits actifs.

La demande de brevet internationale N° WO-A-96/00564 (PRECISION VALVE CORPORATION) fait état d'un composé quaternaire pour aérosol destiné en particulier à être utilisé dans les bombes aérosol de laque pour cheveux et constitué, en plus du principe actif, de :
- 55% au maximum d'un mélange contenant 5 à 50% d'alcool comportant 2 ou 3 atomes de carbone et de 5 à 50% de diméthylether,
- 10 à 45% d'un fluorocarbone sans chlore tel que difluoroéthane ou 1,1,1,2-tetrafluoroéthane,
- 1 à 50% d'eau,
- 1 à 15% de produit actif (typiquement 5%).

La demande de brevet EP-A-343843 décrit une bombe aérosol selon le préambule de la revendication 1.

Les solutions actuelles pour vaporiser un parfum ne permettent pas d'accéder efficacement aux marchés nouveaux du parfumage sophistiqué de l'ambiance qui aurait déjà dû se développer, fort de l'expérience acquise avec les bases parfums élaborées pour les parfums corporels. Les moyens mis en oeuvre pour tenter de remplacer efficacement les CFC sont mal adaptées à la réalisation d'un parfum d'ambiance de haute qualité :
- Les propulseurs liquéfiés les plus usités ont de graves inconvénients. Les hydrocarbures tels que butane, propane ou dimethyléther, sont inflammables, et peu respectueux des bases parfumées de qualité.
- Les gaz comprimés (tels que azote, air pur) posent un problème pour obtenir des pressions constantes en cours d'utilisation, l'ajout nécessaire de solvant volatil pour une bonne pulvérisation est peu compatible avec les parfums de haut de gamme, l'aérosol est peu pratique à l'utilisation : nécessité d'agiter avant l'emploi, de ne pas utiliser tête en bas, ou d'utiliser des valves à billes qui ne sont pas totalement fiables.
- Les vaporisateurs mécaniques à pompe s'avèrent incapables de pulvériser dans l'air ambiant un produit actif liquide composé d'une forte concentration de bases parfumantes dans de l'alcool pour obtenir un fin brouillard indispensable à l'efficacité du parfumage de l'ambiance. De plus le retour d'air à l'intérieur du contenant réduit le temps de conservation pour les produits de qualité.

Le dispositif selon la présente invention a pour objectif de remédier à ces inconvénients. Il permet en effet la réalisation de bombes aérosols diffusant un parfum d'ambiance désodorisant présentant les qualités olfactives d'un parfum corporel, et offrant un grand confort d'utilisation et une grande sécurité d'emploi.

Il est constitué d'un récipient en aluminium 100 % recyclable contenant des bases parfumantes aux normes de la parfumerie en solution dans de l'alcool également de qualité parfumerie, associées à un principe désodorisant à base d'undécylénate d'éthyle, et vaporisées dans l'atmosphère grâce à un propulseur formé d'un mélange de 1,1,1,2-tétrafluoréthane et de fluorure de méthylène, la valve de pulvérisation comportant une prise de gaz additionnelle, c'est-à-dire un orifice sur le corps de valve procurant un apport supplémentaire de propulseur.

Sur les dessins annexés, donnés à titre d'exemple non limitatif d'une des formes de réalisation de l'objet de l'invention:
la figure 1 représente la bombe aérosol en coupe axiale verticale
et la figure 2 est un agrandissement du détail A de la figure 1.

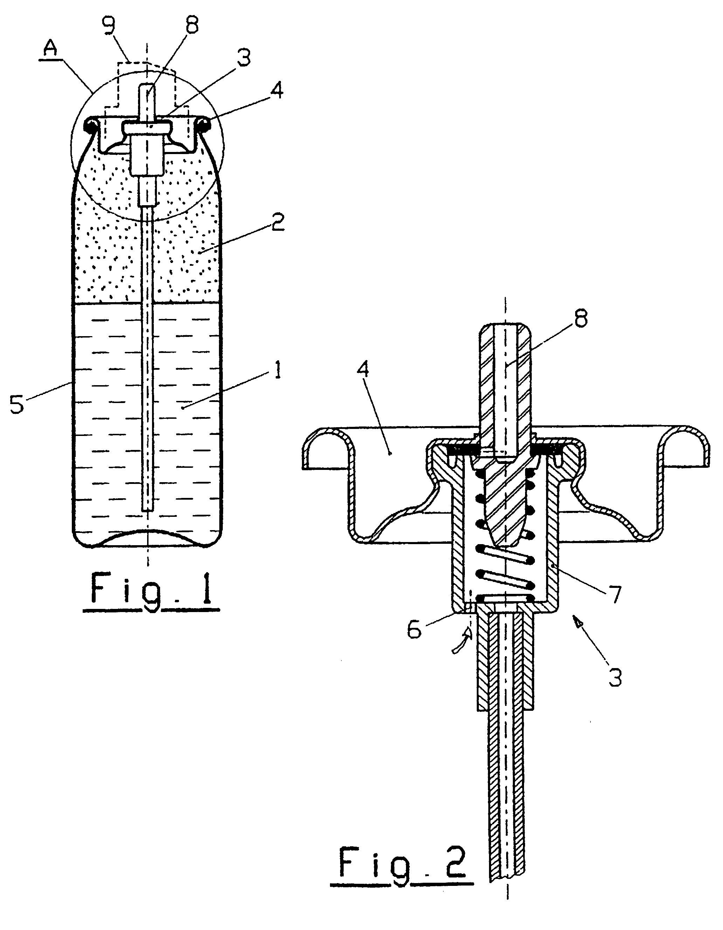

La bombe aérosol objet de la présente invention est remarquable en ce qu'elle associe :
- un principe actif 1 original,
- un nouveau mélange de propulseur 2,
- une valve 3 à prise de gaz additionnelle (figure 2), cette valve étant montée, par l'intermédiaire d'une coupelle 4 de type connu, sur un récipient 5 en aluminium totalement recyclable.

Le principe 1 actif est composé :
- de bases parfumantes à un dosage élevé entre 5 et 20 %, c'est-à-dire une concentration en usage pour les parfums et eaux de toilette corporels. Ces bases étant en tous points respectueuses des normes de la parfumerie et de la cosmétologie, en particulier conformes aux normes de l'Institut Français de Recherches Aromatiques (IFRA) et aux recommandations du Research Institute of Fragrances Materials (RIFM).
- d'alcool éthylique dans une proportion de 70 à 85 %, une faible proportion solvant tel que le propylène glycol pouvant intervenir en Q.S.
- d'un principe désodorisant constitué d'undécylénate d'éthyle (C 11) à un dosage de 0,1 à 0,9 % qui permet sa parfaite incorporation dans des bases parfumantes, le parfumeur ayant à cette concentration la possibilité de l'intégrer dans ses formulations comme un élément constitutif de sa composition. Le C11 est un produit remarquable en ce qu'il est élaboré à partir d'une huile d'origine végétale.

Le mélange de propulseur 2 a été formulé pour répondre à 4 impératifs :
1°) Ne faire appel qu'à des propulseurs de la dernière génération ayant un coefficient nul de destruction de la couche d'ozone.
2°) Offrir un haut niveau de sécurité pour l'homme en étant en particulier pratiquement dénué de nocivité par inhalation, non irritant et non sensibilisant pour la peau.
3°) Etre non inflammable.
4°) Etre compatible avec les parfums les plus raffinés

Un hydrocarbure halogène tel que le 1,1,1,2-tétrafluoréthane ("FORANE 134a" de la Société ATOCHEM) répond à ces impératifs, mais son coût est très élevé et son assez faible tension de vapeur impose des quantités élevées pour obtenir la qualité de dispersion nécessaire aux parfums d'ambiance. Or il est souhaitable de limiter les quantités de propulseur utilisées tout d'abord pour des raisons de coût, mais aussi et surtout pour prendre le moins de risque possible avec l'environnement biologique.

Une formulation nouvelle, faite d'un mélange de propulseurs associés à un type de valve particulier de la bombe aérosol apporte tous les avantages recherchés :
Le propulseur 2 est un mélange nouveau de 1,1,1,2-tétrafluoréthane ("FORANE 134a") pour 60 à 90 % et de fluorure de méthylène, ou difluorométhane ("FORANE 32" de la Société ATOCHEM) pour 10 à 40 %.

Il est à noter que la pression régnant à l'intérieur du récipient 5 est tel que le mélange propulseur se trouve à la fois sous forme liquide, mélangé au principe actif 1 et sous forme gazeuse (2, figure 1) à la partie supérieure partie supérieure dudit récipient.

La valve 3, d'un modèle connu, est de type à prise de gaz additionnelle, c'est-à-dire équipée d'un orifice 6 sur le corps 7 de valve qui procure un apport supplémentaire de propulseur. Elle permet d'obtenir une meilleure diffusion du principe actif 1 avec une pression moins élevée. Le gicleur 8 de la valve 3 sera avantageusement associé à un diffuseur 9 à pulvérisation horizontale.

Dans le cas d'un produit actif 1 constitué de base parfumantes et de désodorisant en solution alcoolique tel que décrit ci-dessus, avec un propulseur 2 constitué de 1,1,1,2-tétrafluoréthane et une valve 3 standard (par exemple modèle K1VT 4 avec diffuseur V04768, marque COSTER), la pression nécessaire est de l'ordre de 4 bars à 20 °C. Pour un volume utile de 60 ml du récipient, la quantité de propulseur nécessaire varie de 15 g à 18 g. La vaporisation est correcte, mais avec des retombées humides, particulièrement à basse température.

En utilisant un mélange composé de 20 % de fluorure de méthylène et de 80 % de 1,1,1,2-tétrafluoréthane %, et en l'associant avec une valve "COSTER" de la série "KVT" avec prise de gaz additionnelle formée d'un trou de 0,3 à 0,5 mm de diamètre, la plus forte pression du fluorure de méthylène permet de réduire la quantité totale de propulseur 2 pour obtenir la pression et la qualité de vidange du boîtier 5 recherchées.

Dans l'exemple ci-après, la quantité de propulseur a pu être réduite à 10 g de mélange, soit moins de 25 % du poids du principe actif :
- Principe actif
   42,7 g ou 51,6 ml
- Mélange gaz propulseur 20/80
   10,0 g ou 8,6 ml
   et donne une pression de 3,2 bars à 20° C qui ne tombe qu'à 2,7 bars à 5 °C et reste maintenue à 5,3 bars à 50 °C ce qui laisse une bonne marge de sécurité, le maximum conseillé étant de ne pas être à plus de 11 à 12 bars à 50 °C.

Avec l'association valve à prise de gaz additionnelle et mélange de propulseurs, la dispersion des micro-gouttelettes atteint une qualité telle, qu'il n'y a pratiquement plus de retombées humides gênantes pour le consommateur.

Par ailleurs la durée de vaporisation est considérablement augmentée, de 50 à 100 % selon le type de valve et le diamètre de l'orifice 6 de prise de gaz, entraînant un nombre de pulvérisations, par exemple de deux à trois secondes, qui peut être pratiquement doublé, donc une amélioration spectaculaire des performances du produit qui peut tirer pleinement parti de sa haute concentration.

De plus, la combinaison de ces trois éléments : produit actif 1, mélange de propulseurs 2 et valve 3 à prise de gaz additionnelle permet aussi de réduire l'inflammabilité au dessous des normes vers lesquelles parait s'acheminer la réglementation pour bénéficier des dérogations à l'étiquetage inflammable, ces normes à notre connaissance devraient être :
- inflammabilité : distance d'ignition < 30 cm
- test d'explosion : durée de vaporisation (s/m3) > 150.

Avec cette nouvelle formulation de propulseurs (80/20 %) et une valve à prise de gaz additionnelle, les tests d'inflammabilité ont montrés une longueur de flamme qui n'a jamais dépassé 24 cm malgré la présence de bases parfumantes inflammables dans de l'alcool à 85 % vol.

Les tests d'explosion en espace clos ont montrés des durées de vaporisations comprises entre 284 s/m3 et 409 s/m3, selon la base parfum utilisée.

Le système aérosol objet de la présente invention permet donc d'obtenir :
- un parfum désodorisant d'ambiance ayant toutes les qualités olfactives que l'on attend d'un parfum corporel,
- une qualité du produit actif et du système propulseur égale du début jusqu'à la fin,
- une action désodorisante réelle grâce à l'undécylénate d'éthyle (C 11) d'origine végétale, n'altérant pas les parfums de qualité,
- une grande sécurité d'utilisation pour la santé, respectant les normes cosmétiques,
- une meilleure sécurité d'emploi au titre de l'inflammabilité,
- une absence de destruction de la couche d'ozone et un meilleur respect de l'environnement biologique
- et surtout une remarquable efficacité d'emploi grâce à la possibilité d'utiliser des principes actifs de hautes concentrations et les disperser parfaitement en micro-gouttelettes très actives dans l'air ambiant sans retombées humides, sans risques de taches
- enfin une augmentation du temps de vaporisation extrêmement profitable à la performance du produit et à l'économie d'utilisation.

L'ensemble de ces avantages appliqués au parfumage de l'ambiance n'avaient jamais été obtenus à ce jour.

## Revendications

1. Bombe aérosol à propulseur liquéfié pour le parfumage d'ambiance, utilisable dans tous les endroits clos et en particulier dans les lieux d'habitation, les lieux de travail et les véhicules, comprenant un récipient avec corps (7) de valve (3) de pulvérisation équipé d'un orifice (6) constituant une prise de gaz additionnelle agencée pour procurer un apport supplémentaire de propulseur au produit vaporisé, le contenu dudit récipient comprenant un principe actif qui comprend des bases parfumantes en solution dans de l'alcool étylique, **caractérisé en ce que** ledit contenu du recipient comprend un principe désodorisant à base d'undécylénate d'éthyle et un mélange propulseur à base de 1,1,1,2 - tétrafluoréthane contenant du fluorure de méthylène et **en ce que** ledit recipient est en aluminium.

2. Bombe aérosol selon la revendication 1, se caractérisant par le fait que le principe actif (1) contient entre 5 et 20 pour cent de bases parfumantes et de 70 à 85 pour cent d'alcool éthylique.

3. Bombe aérosol selon l'une quelconque des revendications précédentes, se caractérisant par le fait que le principe actif (1) contient entre 0,1 et 0,9 pour cent de principe désodorisant à base d'undécylénate d'éthyle d'origine végétale.

4. Bombe aérosol selon l'une quelconque des revendications précédentes, se caractérisant par le fait que le mélange propulseur (2) est composé de 60 à 90 pour cent de 1,1,1,2-tétrafluoréthane et de 10 à 40 pour cent fluorure de méthylène.

5. Bombe aérosol selon la revendication 4, se caractérisant par le fait que le mélange propulseur (2) est composé sensiblement de 80 pour cent de 1,1,1,2-tétrafluoréthane et de 20 pour cent fluorure de méthylène.

6. Bombe aérosol selon l'une quelconque des revendications précédentes, se caractérisant par le fait que le poids du mélange propulseur (2) est inférieur à 25 pour cent du poids du principe actif (1).

## Claims

1. Aerosol canister with liquefied propulsing agent for environment fragrancing, for use in all closed places and in particular in dwellings, places of work and vehicles, comprising a container with body (7) of pulverization valve (3) equipped with an orifice (6) comprising an additional intake arranged to provide an additional input of propulsing agent to the vaporized product, the contents of the aforesaid container holding an active ingredient which includes fragrancing bases in an ethanol alcohol solution,
**characterized in that** said contents of the container include a deodorising active ingredient with ethyl undecylenate base, and a propulsing agent mixture with 1,1,1,2-tetrafluorethane (2) base containing methylene fluoride, and **in that** said container is in aluminium.

2. Aerosol canister according to claim 1, **characterised in that** the active ingredient (1) contains between 5 and 20 percent fragrancing bases and from 70 to 85 percent ethyl alcohol.

3. Aerosol canister according to any one of the aforesaid claims, **characterised in that** the active ingredient (1) contains between 0.1 and 0.9 percent of deodorising active ingredient with ethyl undecylenate base of plant origin.

4. Aerosol canister according to any one of the aforesaid claims, **characterised in that** that the propulsing agent mixture (2) consists of 60 to 90 percent 1,1,1,2-tetrafluorethane and from 10 to 40 percent methylene fluoride.

5. Aerosol canister according to claim 4, **characterised in that** the propulsing agent mixture (2) consists of roughly 80 percent 1,1,1,2-tetrafluorethane and 20 percent methylene fluoride.

6. Aerosol canister according to any one of the aforesaid claims, **characterised in that** the weight of the propulsing agent mixture (2) is less than 25 percent of the weight of the active ingredient (1).

## Patentansprüche

1. Spraydose mit flüssigem Treibmittel zur Parfümierung der Raumluft, verwendbar in allen geschlossenen Räumen, insbesondere in Wohnräumen, Arbeitsräumen und Fahrzeugen, bestehend aus einem Behälter mit dem Körper (7) eines Ventils (3) zur Zerstäubung des Inhalts, das eine Öffnung (6) besitzt, die eine zusätzliche Gaszufuhr darstellt, um dem zerstäubten Produkt zusätzlich Treibmittel zuzuführen, wobei der Inhalt des Behälters einen aktiven Wirkstoff mit parfümierenden Grundsubstanzen enthält, die in Ethylalkohol gelöst sind, **gekennzeichnet dadurch, dass** der genannte Inhalt des Behälters einen deodorierenden Stoff auf Basis von Ethyl-undecylenat und eine Methylenfluorid enthaltene Treibmittelmischung auf Basis von 1,1,1,2-Tetrafluorethan (2) enthält und der genannte Behälter aus Aluminium ist.

2. Spraydose gemäß Anspruch 1, **gekennzeichnet dadurch, dass** der aktive Wirkstoff (1) zwischen 5 und 20 Prozent parfümierende Grundsubstanzen und 70 bis 85 Prozent Ethylalkohol enthält.

3. Spraydose gemäß einem der vorstehenden Ansprüche, **gekennzeichnet dadurch, dass** der aktive Wirkstoff (1) 0,1 bis 0,9 Prozent des deodorierenden Stoffes auf Basis von Ethyl-undecylenat pflanzlichen Ursprungs enthält.

4. Spraydose gemäß einem der vorstehenden Ansprüche, **gekennzeichnet dadurch, dass** die Treibmittelmischung (2) zu 60 bis 90 Prozent aus 1,1,1,2-Tetrafluorethan und zu 10 bis 40 Prozent aus Methylenfluorid besteht.

5. Spraydose gemäß Anspruch 4, **gekennzeichnet dadurch, dass** die Treibmittelmischung (2) im wesentlichen zu 80 Prozent aus 1,1,1,2-Tetrafluorethan und zu 20 Prozent aus Methylenfluorid besteht.

6. Spraydose gemäß einem der vorstehenden Ansprüche, **gekennzeichnet dadurch, dass** das Gewicht der Treibmittelmischung (2) weniger als 25 Prozent des Gewichts des aktiven Wirkstoffs (1) beträgt.
